# EUROPEAN PATENT SPECIFICATION

(11) **EP 0 806 914 B1**
(45) Date of publication and mention of the grant of the patent: **19.09.2001**
(21) Application number: 96900003.3
(22) Date of filing: 03.01.1996
(51) Int. Cl.: A61B 18/08, A61B 18/12

(54) **ELECTROSURGICAL ASPIRATOR COMBINED WITH A PENCIL**
ELEKTROCHIRURGISCHER ABSAUGER KOMBINIERT MIT EINEM HANDSTÜCK
CRAYON ELECTROCHIRURGICAL POURVU D'UN ASPIRATEUR

(30) Priority: 03.02.1995 US 383162
(43) Date of publication of application: 19.11.1997
(73) Proprietor: Sherwood Services AG, 8200 Schaffhausen (CH)
(72) Inventor: GRASSO, Kamala, J., Boulder, CO 80304 (US); KETCHAM, Cliff, Boulder, CO 80303 (US); LEINER, Kevin, Littleton, CO 80123 (US); PACER, Lawrence, K., Littleton, CO 80127 (US); SANCHEZ, Teresa, Broomfield, CO 80020 (US)
(74) Representative: Marsh, Roy David
(86) International application number: IB9600003
(87) International publication number: WO9623448

(56) References cited:
- US-A- 5 154 709
- US-A- 5 224 944

## Description

1. Field of the Invention relates to an aspirator attachment for an electrosurgical pencil having an electrode and more particularly to a passage extending through a semi-rigid body from a distal part to a connector, so that the semi-rigid body resists collapse during handling and use.

2. **Background of the Disclosure** U.S. Patent No. 5,242,442 has an electrosurgical instrument integrated with a suction port consisting of a single small aperture located above the electrode blade. The suction port is unable to trap smoke and heat in an arc of 360° around the electrosurgical electrode. The suction port limits the users field of vision of operative site and the electrosurgical electrode. The '442 patent includes a electrosurgical instrument integrated with a suction port encircling the electrode tip (figure 9). The integrated design allows the suction of blood and saline to be in close proximity to the electrosurgical components of the device which could result in an unwanted conductive electrical energy leakage path. Cleanability and electrical component reliability would not be favorable for that integrated design as compared to a reusable and/or disposable electrosurgical pencil smoke/heat attachment design. Depending on the location of the encircled flat ended nozzle design the user could encounter problems with electrode visibility and trapping or clogging with tissue.

U.S. Patent No. 5,224,944 has a disposable aspirator used with an electrosurgical handpiece. The aspirator includes a continuous flexible tube with the electrode entering the back of the tube. This design could result in the lack of suction due to collapse of or squeezing of the tube at the handpiece and/or tip location. The position at which the electrode enters the tube could create a fluid leak point for conductive blood and/or saline transferring electrosurgical energy from the electrode to the patient or surgeon. The '944 patent includes a fluted holder for the pencil to keep the tube under the pencil. This design could result in the lack of suction due to pinching the soft tube. The '944 patent includes a design for an offset tube connection to the electrode with a cylindrical elastomeric member that connects to the pencil. U.S. patent 5,085,657 the opening at the patient end of the tubular body has a shroud as a separate element being a tube. Alternately, the '657 patent describes a pencil with all of the functions included within a handle and is not a smoke attachment but is a pencil in an integrated unit. U.S. patent 4,562,838 has integrated ducts to direct air for dispersing smoke. U.S. patents 4,719,914 and 4,911,159 has a hollow pencil with a vacuum connection at one end and an electrode and movable nozzle at the other.

U.S. patent 5,055,100 has a tube attached to the pencil with clips; it is not clear that the tube is semi-rigid. The tube is designed to be moved relative to the pencil. U.S. patent 3,906,955 has a pencil with a built in retractable smoke tube exiting beneath and parallel to the electrode. U.S. Patent No. 4,307,720 has a retractable electrode associated with an integral vacuum wand.

U.S. Patent 5,234,428 requires that the passage and the source of vacuum are configured such that the velocity of the air flow into the shroud at the patient end is in a range approximately twice and greater than the expansion velocity of smoke produced by the electrode blade. The '428 has an electrosurgical instrument integrated with a suction port encircling an electrode tip. The integrated design allows the suction of blood and saline to be in close proximity of the electrosurgical components of the device which could result in an unwanted electrical energy leakage path. Cleanability and electrical component reliability would not be helped by that integrated design as compared to an attached disposable electrosurgical pencil smoke/heat design. Depending on the location of the encircled flattened end nozzle design the user could encounter problems with electrode visibility at the operative site and clogging and trapping of removed tissue.

US patent 5, 154, 709 has a vacuum hood attachment for electrosurgical instruments which has a hollow attachment for electrosurgical instruments which has a hollow tubular body open at each end and a side opening for connection to a source of vacuum. One end opening is fitted on the end of an electrosurgical instrument of a pencil- or pen-like hand held construction having an electrocauterizing or electrocuting blade therein. The other open end of said tubular body surrounds the blade along part of the length thereof.

### SUMMARY OF THE INVENTION

An aspirator attachment for an electrosurgical pencil has an axis therethrough. The pencil includes an electrode at a distal end thereof. The aspirator attachment for an electrosurgical pencil most transmits vacuum to remove fluid from a patient's operative site and about the electrode extending from the distal end. A semi-rigid hollow body extends predominately parallel to the axis and the body is contoured to fit coextensively against the elongate electrosurgical pencil. The simi-rigid hollow body has a passage therethrough for the flow of fluid. An opening is disposed at the distal part of the semi-rigid body. The opening is generally about the electrode held in the pencil distal part and the opening faces to be preferably open toward the patient or operative site. The aspirator near the distal part includes a pencil conjugating end aft of the electrode and pencil conjugating end is shaped for generally fluid tight engagement about the distal end of the pencil.

A port is located in the distal part between the pencil conjugating end and its opening toward the patient so that the port is in fluid communication with the passage and the opening. A connector at the end of the semi-rigid body opposite the opening is shaped for fluid communication with the passage and vacuum for removal of fluid. A center section on the semi-rigid body is located substantially adjacent to and coextensive with the electrosurgical pencil. The center section is between the port and the connector for joining them and conjugating with the pencil. An offset transition, of the semi-rigid body adjacent the pencil conjugating end of the pencil, has the passage therethrough for connecting the port and the center section and for alignment therebetween. The passage has a generally uniform cross sectional area extending therethrough from the opening to the connector, so that the flow therethrough is generally constant and so the semi-rigid body resists collapse from vacuum therewithin and during handling and use.

The electrode is preferably coaxially within the opening. The center section of the semi-rigid body at the connector could have an upstanding rib positioned to engage the electrosurgical pencil and retain the generally fluid tight engagement of the opening about the distal end of the pencil and resist relative axial movement between the pencil and the attachment. To resist transverse movement between the pencil and the attachment it is preferred that, a pair of upstanding clips near the connector and on the center section are shaped to engage about the pencil for holding the aspirator attachment semirigid body against the electrosurgical pencil. The pair of clips may be curved to grip the electrosurgical pencil. The pair of clips can be flexible and resiliently spread apart to grip the electrosurgical pencil. A swivel may be attached to the connector to permit relative rotation between the pencil and a hose for connecting to the vacuum. The pencil might include controls along a side thereof and the semi-rigid body is configured to mate with the pencil on a side opposite the controls in a nested engagement for minimizing the overall bulk and resisting rotation between the pencil and the semi-rigid body.

The pencil and the semi-rigid body are preferably elongate having their axial dimensions more than five times their combined thickness dimension. The opening is most preferably scalloped leaving opposite edges of the opening cut away so as to increase visibility, decrease tissue trapping during fluid removal and decrease the temperature of the electrode with fluid flow convection along the electrode. The passage within the center section might have a generally D shaped cross section.

A method for combining an aspirator attachment and an electrosurgical pencil along an axis and an electrode at a distal end thereof so the aspirator attachment transmits vacuum to remove fluid from a patient's operative site and wherein the aspirator attachment has the semi-rigid body with the passage therethrough that extends predominately parallel to the axis, may include steps. Fitting the body coextensively against the elongate electrosurgical pencil is a step. Conjugating the opening at the distal part of the semi-rigid body generally about the electrode facing toward the patient is another step. Locating the opening by a pencil conjugating end aft of the electrode wherein the conjugating end shaped for generally fluid tight engagement about the distal end of the pencil may be a step. Positioning the electrode coaxially within the opening amid scalloped that leave opposite edges of the opening cut away so as to increase visibility of the electrode, decrease tissue trapping during fluid removal and decrease the temperature of the electrode with fluid flow convection along the electrode might be a step. Connecting in fluid communication a port, at the distal part between the pencil conjugating end and its opening, the passage and the opening can be a step. Locating a connector at the end of the semi-rigid body opposite the opening for a hose for connecting to the vacuum could be a step. Placing a center section substantially adjacent to and coextensive with the electrosurgical pencil and between the port and the connector for joining them is a preferred step. Conjugating the center section with the pencil may be a step. Engaging with an upstanding rib, at the center section connector, the electrosurgical pencil for retaining the generally fluid tight engagement of the opening about the distal end of the pencil and for resisting relative axial movement between the pencil and the attachment by configuring the semi-rigid body for mating with the pencil on a side opposite the controls in a nested engagement for minimizing the overall bulk and resisting rotation between the pencil and the semi-rigid body is preferably a step. Aligning the port and an offset transition of the semi-rigid body adjacent the pencil conjugating end of the pencil for connecting the port and the center section can be a step. Extending the passage with a generally uniform cross sectional area therethrough from the opening to the connector, for flow that is generally constant might be a step. Including controls on the pencil along a side of the pencil opposite the mating engagement could be a step.

A method for using and combining an aspirator attachment and electrosurgical pencil has steps including placing the electrode coaxially within the opening to extend axially therefrom toward the operative site. Conjugating the aspirator with the end of the pencil opposite the distal end may be a step. Keeping the conjugated aspirator and pencil together in predominantly parallel relationship to the axis can be a step. Carrying the aspirator opposite the pencil controls for permitting undisturbed access to the controls might be a step. Locating the opening coaxially about the electrode at the distal end offset and parallel to the aspirator center section might be a step. Connecting to the opening with the parallel aspirator through a port in the opening with an offset transition extending the passage therebetween could be a step. Transmitting vacuum from a source through the passage, the offset transition and the opening to the environs of the operative site is a preferred step. Keeping the conjugated aspirator and pencil transversely together with the preferred steps of securing the aspirator to the pencil with the pair of clips and preventing the axial movement therebetween with an upstanding rib engaging the pencil at its end opposite the distal end.

### BRIEF DESCRIPTION OF THE DRAWINGS

Figure 1 is a perspective view of an aspirator attachment for an electrosurgical pencil showing them coupled together for use.

Figure 2 is a view in cross section as would be seen along line 2-2 of Figure 1 showing the conjugation between the aspirator attachment for an electrosurgical pencil.

Figure 3 is a cross sectional view of Figure 1 of the proximal end of the aspirator attachment for an electrosurgical pencil showing a swivel connector and the particular interengagement of the rib and the pencil.

Figure 4 is an enlarged cross sectional view of the distal part of the aspirator attachment for an electrosurgical pencil showing an offset transition and its particular interengagement with the distal end of the pencil so that the electrode extends therefrom within an opening.

Figure 5 is an enlarged partial perspective view of the scalloped opposite edges of the aspirator opening to increase electrode visibility, decrease tissue trapping during fluid removal and decrease the temperature of the electrode with fluid flow convection along the electrode.

Figure 6 is an enlarged cross sectional view along line 6-6 of Figure 1 of the center section of the aspirator attachment and in particular the D shaped cross section thereof.

### DETAILED DESCRIPTION OF THE INVENTION

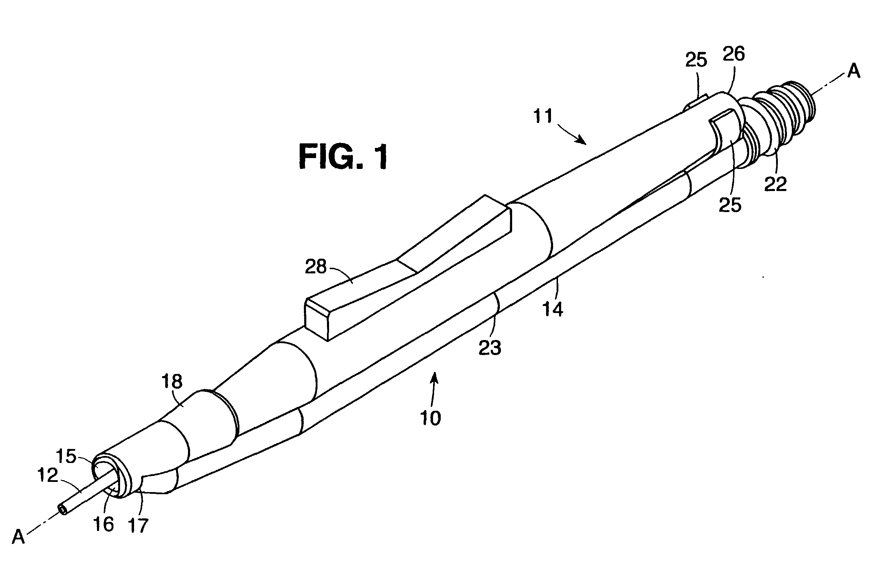

An aspirator attachment 10 for an electrosurgical pencil 11 shown in Figure 1 has an axis "A". The pencil 11 shown in lighter lines with an electrode 12 extending from a distal end 13 thereof is manufactured by Valleylab, Boulder Colorado as model numbers E2515, E2516 or E2550. The aspirator attachment 10 is for transmitting vacuum to remove fluid from a patient's operative site (not shown). A semi-rigid hollow body 14 extends predominately parallel to the axis "A" and contoured to fit coextensively against the elongate electrosurgical pencil 11. The semi-rigid hollow body 14 has a passage 15 therethrough with an opening 16 at the distal part 17 of the semi-rigid hollow body 14. The opening 16 generally about the electrode 12 faces toward the patient.

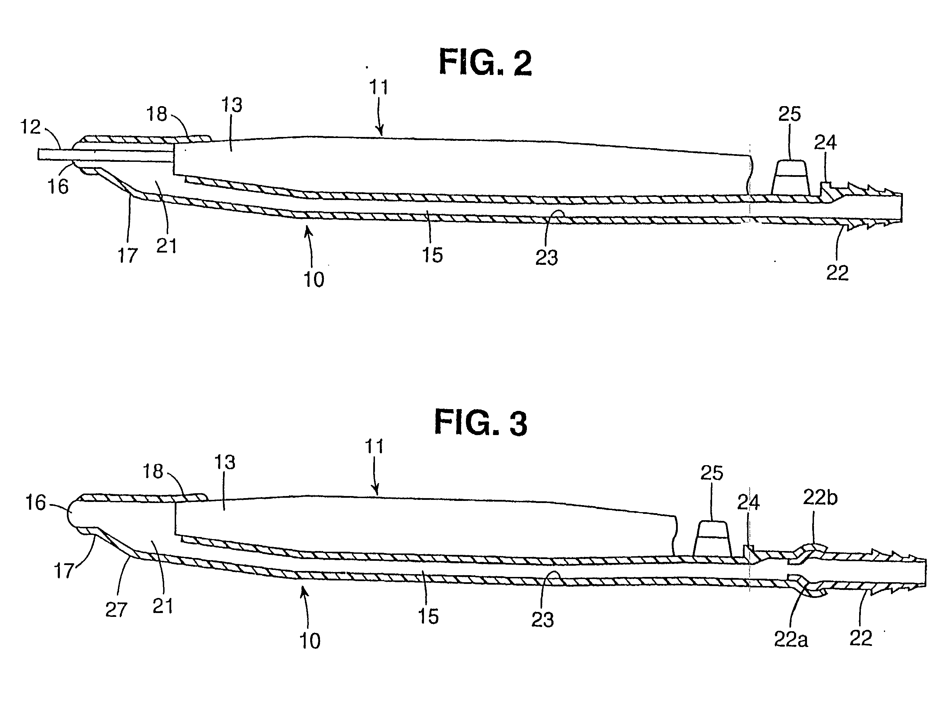

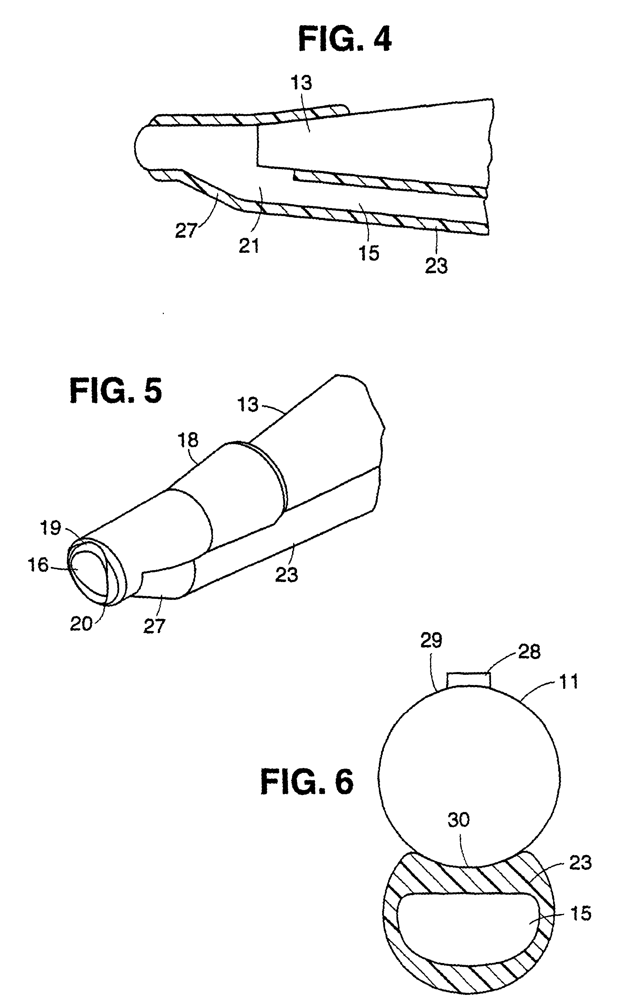

The distal part 17 includes a pencil conjugating end 18, as best illustrated in Figure 2, aft of the electrode 12 and shaped for generally fluid tight engagement about the distal end 13 of the pencil 11. The cross section of Figure 2 is only one longitudinal half of the semirigid hollow body 14 and the complete assembly is formed of two components that are mirror images of one another made of plastic polymer such as styrene or liquid crystal polymer and ultrasonically welded into a sealed unit that has the fluid tight passage 15. The electrode 12 is coaxially positioned within the opening 16. The opening 16 is scalloped leaving opposite edges 19 and 20 of the opening 16 cut away so as to increase visibility, decrease tissue trapping during fluid removal and decrease the temperature of the electrode 12 with fluid flow convection along the electrode 12, see Figure 5.

A port 21 is in the distal part 17 between the pencil conjugating end 18 and its opening 16 facing the patient. The port 21 is in fluid communication with the passage 15 and the opening 16. A connector 22 at the end of the semi-rigid hollow body 14 is located opposite the opening 16, see Figure 2. The connector 22 is shaped for fluid communication with the passage 15 and vacuum for removal of fluid (not shown). A swivel 22b, in Figure 3, attaches to a ball shaped connector 22a and permits relative rotation between the pencil 11 and a hose (not shown) for connecting to the vacuum as per Figures 2 and 3. A center section 23 on the semi-rigid body 14 is located substantially adjacent to and coextensive with the electrosurgical pencil 11. The center section 23 between the port 21 and the connector 22 or 22a joins them and is shaped to conjugate with the pencil 11. The center section 23 of the semi-rigid hollow body 14 at the connector 22 or 22a has an upstanding rib 24 shown in Figures 2 and 3; the rib 24 is positioned to engage the electrosurgical pencil 11 and retain the generally fluid tight engagement of the conjugating end 18 about the distal end 13 of the pencil 11. The rib 24 resists relative axial movement between the pencil and the aspirator attachment 10. To resist transverse movement between the pencil 11 and the aspirator attachment 10 it is preferred that, a pair of upstanding clips 25 in figures 1, 2, and 3 near the connector and on the center section 23 are shaped to engage about the pencil 11 at its proximal end 26 for holding the aspirator attachment 10 and the semirigid hollow body 14 thereof against the electrosurgical pencil 11. The pair of clips 25 are curved to grip the electrosurgical pencil 11. The pair of clips 25 can be flexible and resiliently spread apart to enhance the grip of the electrosurgical pencil 11. The pencil 11 and the semi-rigid hollow body 14 are elongate, for ergonomics, having their axial dimensions roughly more than five times their combined thickness dimension.

An offset transition 27 of the semi-rigid hollow body 14 is located adjacent the pencil 11 conjugating end 18 as best shown in the enlarged cross section of Figure 4. The offset transition 27 includes the passage 15 therethrough for connecting the port 21 and the center section 23 and for alignment therebetween. The passage 15 has a generally uniform cross sectional area extending therethrough from the opening 16 to the connector 22 or 22a, so that the flow therethrough is generally constant. As seen in Figure 6, the passage 15 within the center section 23 has a generally "D" shaped cross section and the pencil 11 includes controls 28 along a outside thereof. The semi-rigid hollow body 14 is configured to mate with the pencil 11 on an inside 30 opposite the controls 28 in a nested engagement for minimizing the overall bulk and resisting rotation between the pencil 11 and the semi-rigid hollow body 14.

A method for combining aspirator attachment 10 and electrosurgical pencil 11 having axis "A" requires the pencil 11 with its electrode 12 at the distal end 13 thereof. The aspirator attachment 10 transmits vacuum from a source that is not shown as it is not essential to the method but hospitals have vacuum systems including bioburden filtering means to remove air and eliminate hazardous waste products. Vacuum removes fluid including smoke from the operative site and the aspirator attachment 10 has its semi-rigid hollow body 14 with passage 15 therethrough extending predominately parallel to axis "A". The method has steps including fitting the body 14 coextensively against the elongate eiectrosurgical pencil 11 and conjugating the opening 16 at the distal part 17 of the semi-rigid hollow body 14 generally about electrode 12 to face toward the patient. Locating the opening 16 with the pencil conjugating end 18 aft of the electrode 12 is a step of the method. Conjugating the end 18 shaped for generally fluid tight engagement about the distal end 13 of the pencil 11 is a method step the results of which are shown in the cross sectional views of Figures 2, 3 and 4. The step of positioning electrode 12 coaxially within opening 16 having scalloped edges 19 and 20 leave opposite edges of the opening 16 cut away so as to increase visibility, decrease tissue trapping during fluid removal and decrease the temperature of electrode 12 with fluid flow convection therealong is in the method. Connecting in fluid communication port 21, at distal part 17 between pencil conjugating end 18 and opening 16, passage 15 and opening 16 is a step. Another step of the method includes locating connector 22 or 22a at the end of the semi-rigid hollow body 14 opposite opening 16 for connecting to the vacuum. Placing center section 23 substantially adjacent to and coextensive with electrosurgical pencil 11 and between port 21 and connector 22 or 22a for joining them is a further step in combining them. Then the step of conjugating center section 23 with pencil 11 is performed. Thus engaging upstanding rib 24, at center section 23 near connector 22 or 22a, for retaining electrosurgical pencil in the generally fluid tight engagement with opening 16 about distal end 13 of pencil 11 and for resisting relative axial movement between pencil 11 and aspirator attachment 10 by configuring semi-rigid hollow body 14 for mating with pencil 11 on inside 30 opposite controls 28 in a nested engagement for minimizing the overall bulk and resisting rotation between pencil 11 and semi-rigid hollow body 14 to realize the method. Aligning port 21 and offset transition 27 of semi-rigid hollow body 14 adjacent pencil conjugating end 18 for connecting port 21 and center section 23 is a step. Thereby extending passage 15 with a generally uniform cross sectional area therethrough from opening 16 to connector 22 or 22a, for flow generally constant through the combination is a step. Including controls 28 on pencil 11 along outside 29 opposite the mating engagement is a step.

A method for using and combining aspirator attachment 10 and electrosurgical pencil 11 having axis "A", includes electrode 12 extending from distal end 13 and aspirator attachment 10 for transmitting vacuum to remove fluid through opening 16 near the operative site. The aspirator attachment 10 has its semi-rigid hollow body 14 with passage 15 therethrough extending predominately parallel to the axis "A". The method has steps including placing electrode 12 coaxially within opening 16 to extend axially therefrom toward the operative site. Conjugating the aspirator attachment 10 with the proximal end 26 of the pencil 11 opposite distal end 13 and keeping conjugated aspirator attachment 10 and pencil 11 together in predominantly parallel relationship to the axis "A" are steps. Carrying the aspirator attachment 10 opposite pencil controls 28 for permitting undisturbed access to controls 28 and locating opening 16 coaxially about electrode 12 at distal part 17 offset and parallel to the aspirator center section 23 are steps. The method includes the steps of connecting to opening 16 with the parallel aspirator attachment 10 through port 21 with offset transition 27 extending passage 15 therebetween and transmitting vacuum from a source through passage 15, offset transition 27 and opening 16 to the environs of the operative site.

The method keeps the conjugated aspirator attachment 10 and pencil 11 together transversely with the steps of securing the aspirator attachment 10 to the pencil 11 with clips 25 and preventing the axial movement therebetween with upstanding rib 24 engaging pencil 11 at its proximal end 26 opposite distal end 13.

## Claims

1. An aspirator attachment (10) for an electrosurgical pencil (11) having a long axis and convex cross-section, the pencil with an electrode (12) at a distal end (13) thereof, the aspirator attachment (10) for transmitting vacuum to remove fluid from a patient's operative site comprising:
said electrosurgical pencil;
a semi-rigid hollow body (14) having a passage (15) therethrough;
an opening (16) at the distal part (17) of the semi-rigid body, the opening (16) generally about the electrode (12) and facing toward the patient, the distal part (17) including a pencil conjugating end (18) aft of the electrode (12) and shaped for generally fluid tight engagement about the distal end (13) of the pencil;
a port (21) in the distal part (17) between the pencil conjugating end (18) and its opening (16) toward the patient, the port (21) in fluid communication with the passage (15) and the opening (16);
a connector (22) at the end of the semi-rigid body opposite the opening (16), the connector (22) shaped for fluid communication with the passage (15) and vacuum for removal of fluid;
a center section on the semi-rigid body located substantially adjacent to the electrosurgical pencil (11), the center section between the port (21) and the connector (22) for joining them and conjugating with the pencil;
an offset transition (27) of the semi-rigid body adjacent the pencil conjugating end (18) , the offset transition (27) with the passage (15) therethrough for connecting the port (21) and the center section and for alignment therebetween, and
the passage (15) having a generally uniform cross sectional area extending therethrough from the opening (16) to the connector (22), so that the flow therethrough is generally constant and so the semi-rigid body resists collapse from vacuum therewithin and during handling and use,
**characterised in that**,
the semi-rigid body extends predominately parallel to the axis and is contoured with a concave portion to fit coextensively against the elongate electrosurgical pencil (11); the semi-rigid body having a length corresponding to that of the pencil.

2. An aspirator attachment as claimed in claim 1, attached to an electrosurgical pencil.

3. The aspirator attachment (10) for an electrosurgical pencil (11) of claim 2 wherein the electrode (12) is coaxially within the opening (16).

4. The aspirator attachment (10) for an electrosurgical pencil (11) of claim 2 or 3 wherein the center section of the semi-rigid body at the connector (22) has an upstanding rib (24) positioned to engage the electrosurgical pencil (11) and retain the generally fluid tight engagement of the opening (16) about the distal end (13) of the pencil and resist relative axial movement between the pencil and the attachment.

5. The aspirator attachment (10) for an electrosurgical pencil 11 of claim 2, 3 or 4 wherein a swivel (22a) attached to the connector (22) permits relative rotation between the pencil and a hose for connecting to the vacuum.

6. The aspirator attachment (10) for an electrosurgical pencil (11) of at least one of the preceding claims wherein the pencil includes controls along a side thereof and the semi-rigid body is configured to mate with the pencil on a side opposite the controls in a nested engagement for minimizing the overall bulk and resisting rotation between the pencil and the semi-rigid body.

7. The aspirator attachment (10) for an electrosurgical pencil 11 of at least one of the preceding claims wherein the pencil and the semi-rigid body are elongate having their axial dimensions more than five times their combined thickness dimension.

8. The aspirator attachment (10) for an electrosurgical pencil (11) of at least one of the preceding claims wherein the opening (16) is scalloped leaving opposite edges (19) and (20) of the opening (16) cut away so as to increase visibility, decrease tissue trapping during fluid removal and decrease the temperature of the electrode (12) with fluid flow convection along the electrode (12).

9. The aspirator attachment (10) for an electrosurgical pencil (11) of at least one of the preceding claims wherein the passage (15) within the center section has a generally D shaped cross section.

10. The aspirator attachment (10) for an electrosurgical pencil (11) of at least one of the preceding claims , wherein a pair of upstanding clips (25) near the connector (22) and an the center (25) section are shaped to engage about the pencil for holding the aspirator attachment (10) semi-rigid body against the electrosurgical pencil (11).

11. The aspirator attachment (10) for an electrosurgical pencil (11) of claim 10 wherein the pair of clips are curved to grip the electrosurgical pencil (11).

12. The aspirator attachment (10) for an electrosurgical pencil (11) of claim 10 or 11 wherein the pair of clips are flexible and resiliently spread apart to grip the electrosurgical pencil (11).

## Patentansprüche

1. Eine Aspiratorbefestigung (10) für einen elektrochirurgischen Stift (11) weist eine lange Achse und einen konvexen Querschnitt auf, der Stift weist eine Elektrode (12) an seinem distalen Ende (13) auf, die Aspiratorbefestigung (10) zum Übertragen von Vakuum, um Fluid von einer operativen Stelle eines Patienten zu beseitigen, umfasst:
den elektrochirurgischen Stift (11);
einen halbstarren, hohlen Körper (14) mit einem Durchgang (15) hierdurch;
eine Öffnung (16) an dem distalen Teil (17) des halbstarren Körpers, die Öffnung (16) befindet sich im Allgemeinen um die Elektrode (12) und zeigt zu dem Patienten, das distale Teil (17) schließt ein stiftkonjugierendes Ende (18) hinter der Elektrode (12) ein und ist zum im Allgemeinen fluiddichten Eingriff um das distale Ende (13) des Stiftes geformt;
einen Anschluss (21) in dem distalen Teil (17) zwischen dem stiftkonjugierenden Ende (18) und seiner Öffnung (16) zu dem Patienten, der Anschluss (21) ist in fluider Verbindung mit dem Durchgang (15) und der Öffnung (16) ;
einen Verbinder (22) an dem Ende des halbstarren Körpers gegenüber der Öffnung (16), der Verbinder (22) ist geformt zur fluiden Verbindung mit dem Durchgang (15) und dem Vakuum zur Beseitigung von Fluid;
einen mittleren Abschnitt auf dem halbstarren Körper, der sich im Wesentlichen neben dem elektrochirurgischen Stift (11) befindet, der mittlere Abschnitt dient der Verbindung zwischen dem Anschluss (21) und dem Verbinder (22) und dem Konjugieren mit dem Stift;
einen Ausgleichsübergang (27) des halbstarren Körpers neben dem stiftkonjugierenden Ende (18), der Ausgleichsübergang (27) mit dem Durchgang (15) hierdurch dient der Verbindung des Anschlusses (21) und des mittleren Abschnittes und der Ausrichtung dazwischen, und wobei
der Durchgang (15) eine im Allgemeinen gleichmäßige Querschnittsflache aufweist, die sich hierdurch von der Öffnung (16) zu dem Verbinder (22) erstreckt, so dass der Fluss hierdurch im Allgemeinen konstant ist und so dass der halbstarre Körper einem Kollabieren durch das Vakuum und während der Handhabung und des Gebrauchs widersteht,
**dadurch gekennzeichnet, dass**
der halbstarre Körper sich überwiegend parallel zu der Achse erstreckt und eine Kontur mit einem konkaven Abschnitt aufweist, um sich koextensiv gegen den länglichen, elektrochirurgischen Stift (11) anzupassen; der halbstarre Körper eine Länge entsprechend der des Stiftes aufweist,

2. Eine Aspiratorbefestigung nach Anspruch 1, die an einen elektrochirurgischen Stift angebracht ist.

3. Aspiratorbefestigung (10) für einen elektrochirurgischen Stift (11) nach Anspruch 2, wobei die Elektrode (12) koaxial innerhalb der Öffnung (16) angeordnet ist.

4. Aspiratorbefestigung (10) für einen elektrochirurgischen Stift (11) nach Anspruch 2 oder 3, wobei der mittlere Abschnitt des halbstarren Körpers an dem Verbinder (22) eine aufrechtstehende Rippe (24) aufweist, die derart positioniert ist, um mit dem elektrochirurgischen Stift (11) im Eingriff zu sein, und die den im Allgemeinen fluiddichten Eingriff der Öffnung (16) um das distale Ende (13) des Stiftes aufrechterhält und einer relativen axialen Bewegung zwischen dem Stift und der Befestigung widersteht.

5. Aspiratorbefestigung (10) für einen elektrochirurgischen Stift (11) nach Anspruch 2, 3 oder 4, wobei ein Drehlager (22a), das an dem Verbinder (22) angebracht ist, eine relative Drehung zwischen dem Stift und einem Schlauch zum Verbinden mit dem Vakuum ermöglicht.

6. Aspiratorbefestigung (10) für einen elektrochirurgischen Stift (11) nach zumindest einem der vorhergehenden Ansprüche, wobei der Stift eine Steuereinrichtung entlang einer Seite desselben umfasst und der halbstarre Körper derart konfiguriert ist, um mit dem Stift an einer Seite gegenüber der Steuereinrichtung in einem verschachtelten Eingriff zusammenzupassen zum Minimieren des gesamten Volumens und zum Widerstehen einer Drehung zwischen dem Stift und dem halbstarren Körper.

7. Aspiratorbefestigung (10) für einen elektrochirurgischen Stift (11) nach zumindest einem der vorhergehenden Ansprüche, wobei der Stift und der halbstarre Körper länglich sind und ihre axialen Abmessungen mehr als das Fünffache ihrer vereinten Dickenabmessung betragen.

8. Aspiratorbefestigung (10) für einen elektrochirurgischen Stift (11) nach zumindest einem der vorhergehenden Ansprüche, wobei die Öffnung (16) ausgebogen ist, so dass die gegenüberliegenden Ränder (19 und 20) der Öffnung (16) weggeschnitten sind, um so das Sichtvermögen zu erhöhen, das Einfangen von Gewebe während der Fluidbeseitigung zu verringern und die Temperatur der Elektrode (12) durch Konvektion des Fluidflusses entlang der Elektrode (12) zu reduzieren.

9. Aspiratorbefestigung (10) für einen elektrochirurgischen Stift (11) nach zumindest einem der vorangegangenen Ansprüche, wobei der Durchgang (15) innerhalb des mittleren Abschnittes einen im Allgemeinen D-förmigen Querschnitt aufweist.

10. Aspiratorbefestigung (10) für einen elektrochirurgischen Stift (11) nach zumindest einem der vorhergehenden Ansprüche, wobei ein Paar von aufrechtstehenden Clips (25) in der Nähe des Verbinders (22) und an dem mittleren Abschnitt derart geformt sind, dass sie um den Stift im Eingriff sind zum Halten des halbstarren Körpers der Aspiratorbefestigung (10) gegen den elektrochirurgischen Stift (11).

11. Aspiratorbefestigung (10) für einen elektrochirurgischen Stift (11) nach Anspruch 10, wobei das Paar von Clips gekrümmt ist, um den elektrochirurgischen Stift (11) zu greifen.

12. Aspiratorbefestigung (10) für einen elektrochirurgischen Stift (11) nach Anspruch 10 oder 11, wobei das Paar von Clips elastisch ist und sich elastisch aufweitet, um den elektrochirurgischen Stift (11) zu greifen.

## Revendications

1. Accessoire d'aspiration (10) pour un crayon électrochirurgical (11) ayant un axe long et une section transversale convexe, le crayon avec une électrode (12) à une extrémité distale (13) de celui-ci, l'accessoire d'aspiration (10) pour transmettre un vide afin de retirer du fluide d'un site opératoire d'un patient comprenant :
- ledit crayon electrochirurgical ;
- un corps creux semi-rigide (14) ayant un passage (15) à travers celui-ci ;
- une ouverture (16) à la partie distale (17) du corps semi-rigide, l'ouverture (16) étant généralement autour de l'électrode (12) et étant orientée vers le patient, la partie distale (17) incluant une extrémité de conjugaison de crayon (18) en aval de l'électrode (12) et configurée pour un engagement généralement étanche au fluide autour de l'extrémité distale (13) du crayon ;
- un orifice (21) dans la partie distale (17) entre l'extrémité de conjugaison de crayon (18) et son ouverture (16) vers le patient, l'orifice (21) étant en communication de fluide avec le passage (15) et l'ouverture (16) ;
- un connecteur (22) à l'extrémité du corps semi-rigide opposée à l'ouverture (16), le connecteur (22) étant configuré pour une communication de fluide avec le passage (15) et le vide pour le retrait du fluide ;
- une section centrale sur le corps semi-rigide localisée d'une manière sensiblement adjacente au crayon électrochirurgical (11), la section centrale entre l'orifice (21) et le connecteur (22) pour les assembler et combiner avec le crayon ;
- une transition décalée (27) du corps semi-rigide adjacente à l'extrémité de conjugaison de crayon (18), la transition décalée (27) avec le passage (15) à travers celle-ci pour relier l'orifice (21) et la section centrale et pour l'alignement entre ceux-ci, et
- le passage (15) ayant une zone en section transversale généralement uniforme s'étendant à travers celui-ci. à partir de l'ouverture (16) au connecteur (22), de telle sorte que le flux à travers celui-ci est généralement constant et ainsi le corps semi-rigide résiste à un affaissement par le vide dans celui-ci et pendant la manipulation et l'utilisation,
**caractérisé en ce que**
- le corps semi-rigide s'étend sensiblement parallèlement à l'axe et présente un contour avec une portion concave pour s'adapter d'une manière coextensive contre le crayon électrochirurgical oblong (11) ; le corps semi-rigide ayant une longueur correspondant à celle du crayon.

2. Accessoire d'aspiration selon la revendication 1, fixé à un crayon électrochirurgical.

3. Accessoire d'aspiration (10) pour un crayon électrochirurgical (11) selon la revendication 2, où l'électrode (12) se situe coaxialement dans l'ouverture (16).

4. Accessoire d'aspiration (10) pour un crayon électrochirurgical (11) selon la revendication 2 ou 3, où la section centrale du corps semi-rigide au connecteur (22) présente une nervure érigée (24) positionnée pour venir en prise avec le crayon électrochirurgical (11) et pour conserver l'engagement généralement étanche au fluide de l'ouverture (16) autour de l'extrémité distale (i3) du crayon et pour résister à un déplacement axial relatif entre le crayon et l'accessoire.

5. Accessoire d'aspiration (10) pour un crayon électrochirurgical (11) selon la revendication 2, 3 ou 4, où un pivot (22a) fixé au connecteur (22) permet une rotation relative entre le crayon et un tuyau souple pour la connexion au vide.

6. Accessoire d'aspiration (10) pour un crayon électrochirurgical (11) selon au moins l'une des revendications précédentes, où le crayon comprend des organes de réglage le long d'un côté de celui-ci, et le corps semi-rigide est configure pour s'adapter au crayon sur un côté opposé aux organes de réglage selon un engagement d'emboîtement afin de réduire à un minimum l'ensemble de la masse et de résister à la rotation entre le crayon et le corps semi-rigide.

7. Accessoire d'aspiration (10) pour un crayon électrochirurgical (11) selon au moins l'une des revendications précédentes, où le crayon et le corps semi-rigide sont oblongs, leurs dimensions axiales représentant plus que cinq fois leur dimension en épaisseur combinée.

8. Accessoire d'aspiration (10) pour un crayon électrochirurgical (11) selon au moins l'une des revendications précédentes, où l'ouverture (16) est échancrée, en laissant des bords opposés (19) et (20) de l'ouverture (16) découpés pour augmenter la visibilité, pour réduire un accrochage du tissu pendant le retrait du fluide et pour diminuer la température de l'électrode (12) avec la convection du flux de fluide le long de l'électrode.

9. Accessoire d'aspiration (10) pour un crayon électrochirurgical (11) selon au moins l'une des revendications précédentes, où le passage (15) dans la section centrale a une section transversale généralement en forme de D.

10. Accessoire d'aspiration (10) pour un crayon électrochirurgical (11) selon au moins l'une des revendications précédentes, où une paire de pinces érigées (25) près du connecteur (22) et une section centrale (25) sont configurées pour venir en prise avec le crayon pour tenir le corps semi-rigide de l'accessoire d'aspiration (10) contre le crayon électrochirurgical (11).

11. Accessoire d'aspiration (10) pour un crayon électrochirurgical (11) selon la revendication 10, où la paire de pinces est courbée pour saisir le crayon électrochirurgical (11).

12. Accessoire d'aspiration (10) pour un crayon électrochirurgical (11) selon la revendication 10 ou 11, ou la paire de pinces est flexible et est écartée élastiquement pour saisir le crayon électrochirurgical (11).
